(19) Europäisches Patentamt
European Patent Office
Office européen des brevets

(11) Publication number: 0 105 919
B1

(12) # EUROPEAN PATENT SPECIFICATION

(45) Date of publication of patent specification: **08.07.87**

(51) Int. Cl.$^4$: **C 07 D 209/42,** A 61 K 31/405

(21) Application number: **83901683.9**

(22) Date of filing: **25.04.83**

(86) International application number: **PCT/US83/00597**

(87) International publication number: **WO 83/03828 10.11.83 Gazette 83/26**

(54) **1-CARBOXYALKANOYLPERHYDROINDOLE-2-CARBOXYLIC ACIDS AND DERIVATIVES.**

(30) Priority: **26.04.82 US 371700**

(43) Date of publication of application: **25.04.84 Bulletin 84/17**

(45) Publication of the grant of the patent: **08.07.87 Bulletin 87/28**

(84) Designated Contracting States: **BE CH DE FR GB LI LU NL SE**

(56) References cited:
**EP-A-0 031 741**
**EP-A-0 037 231**
**EP-A-0 049 658**
**EP-A-0 050 850**

**The file contains technical information submitted after the application was filed and not included in this specification**

(73) Proprietor: **CIBA-GEIGY AG**
**Klybeckstrasse 141**
**CH-4002 Basel (CH)**

(72) Inventor: **GRUENFELD, Norbert**
**19 Sparrow Circle**
**White Plains, NY 10605 (US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 105 919 B1

**Description**

European patent application 31 741 discloses a variety of bicyclic nitrogen containing heterocyclic carboxylic acid derivatives, the nitrogen atom of those being substituted by the grouping

$$-CO-\underset{\displaystyle R}{\underset{|}{CH}}-(CH_2)_q-Z$$

wherein R is hydrogen, methyl or benzyl, q is zero, one or two and Z is carboxy, cyano, hydroxy, mercapto or amino. These compounds are described to have antihypertensive properties, in particular by inhibiting the angiotensin converting enzyme. As a sidechain predominantly the grouping

$$-CO-\underset{\displaystyle CH_3}{\underset{|}{CH}}-CH_2-SH$$

is used which is well known from captopril. Pharmacological data are presented for five different heterocycles substituted on the nitrogen by said mercapto side chain. Additionally there are data for the only compound disclosed having a carboxy group in the side chain.

The present invention concerns new 1-carboxy-(alkanoyl or aralkanoyl)perhydroindole-2-carboxylic acids of the formula I

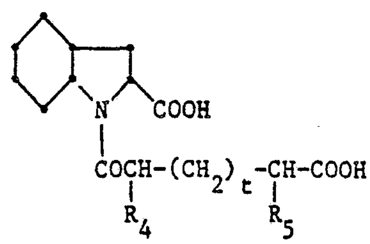

(I)

wherein $R_4$ represents hydrogen or lower alkyl; $R_5$ represents lower alkyl; or $R_5$ represents lower alkyl substituted by aryl in which aryl represents phenyl or phenyl substituted by lower alkyl, lower alkoxy, trifluoromethyl or halogen; t represents 0 or 1; the unsubstituted amides and mono- or di-lower alkylamides thereof; lower alkylene amides thereof, wherein the lower alkylene group together with the amide nitrogen forms a 5-, 6- or 7-membered ring, or said lower alkylene amides substituted on the ring by hydroxy-(lower)alkyl or by lower alkanoyloxy-(lower)alkyl; α-(lower)-carboalkoxy- or α-carboxy-substituted lower alkylamides thereof; α-(lower)carboalkoxy- or α-carboxy-substituted aryl-(lower) alkylamides in which aryl represents phenyl or 3-indolyl; (amino or acylamino)-(lower) alkylamides thereof; lower alkyl esters thereof; (amino, mono- or di-lower alkylamino, carboxy or lower carboalkoxy)-substituted lower alkyl esters thereof; aryl-(lower) alkyl esters thereof in which aryl represents phenyl or pyridyl; lower alkanoyloxy-(lower) alkyl esters thereof; phthalidyl esters thereof; (hydroxy, lower alkanoyloxy, or lower alkoxy)-substituted (lower)alkoxymethyl esters thereof; bicycloalkyloxycarbonyl-(lower) alkyl esters thereof having up to 10 carbon atoms in the bicycloalkyl group; or lower alkoxycarbonyloxy-(lower) alkyl esters thereof; or pharmaceutically acceptable salts thereof, process for their manufacture, pharmaceutical compositions containing these compounds and these compounds for use in therapeutic applications.

A preferred embodiment relates to the perhydroindoles of formula I, preferably the *cis* fused perhydroindole-2S-epimers, wherein $R_4$ represents hydrogen or lower alkyl of up to 4 carbon atoms; $R_5$ represents lower alkyl of up to 4 carbon atoms; or $R_5$ represents lower alkyl of up to 4 carbon atoms substituted by aryl in which aryl represents phenyl or phenyl monosubstituted by lower alkyl, lower alkoxy or halogen; t represents 0 or 1; lower alkyl esters, e.g. the methyl, ethyl, n- or i-propyl or -butyl esters; substituted lower alkyl esters e.g. the ω-amino, ω-mono- or N-dimethylamino, α-carboxy or α-carbethoxy-(ethyl, propyl or butyl) esters; aryl(lower) alkyl esters, e.g. benzyl, (methyl-, methoxy-, chloro-)substituted benzyl, and pyridylmethyl esters; lower alkanoyloxy-(lower) alkyl esters, e.g. pivaloyloxymethyl esters; 3-phthalidyl and (methyl-, methoxy-, chloro-)substituted 3-phthalidyl esters, derived from the corresponding 3-hydroxyphthalides, (hydroxy, lower alkanoyloxy-, lower alkoxy-)substituted lower alkoxymethyl esters, e.g, β-(hydroxy-, acetyloxy-, methoxy-) ethoxymethyl esters; bicycloalkyloxy-carbonyl-(lower) alkyl esters, e.g. those derived from bicyclic monoterpenoid alcohols, such as unsubstituted or lower alkyl substituted bicyclo [2,2,1]heptyloxycarbonyl-(lower)alkyl esters, advantageously bornyloxycarbonylmethyl esters; or 1-(lower alkoxycarbonyloxy)-(lower) alkyl esters, e.g. 1-(methoxy-, ethoxy- or propoxycarbonyloxy)-methyl, ethyl or propyl esters thereof; and pharmaceutically acceptable salts thereof.

Preferred are the said perhydroindoles wherein t is 1.

The mono ester or amide derivatives of the compounds of formula I are those at either of the two carboxyl groups. Preferred are the mono ester or amide derivatives with a free perhydro-2-indole carboxyl group.

A preferred group is represented by the compounds of formula I, preferably the *cis* fused perhydroindole-2S-epimers, wherein $R_4$ represents hydrogen or lower alkyl of up to 4 carbon atoms; $R_5$ represents lower alkyl of up to 4 carbon atoms; or $R_5$ represents lower alkyl of up to 4 carbon atoms substituted by aryl in which aryl represents phenyl or phenyl mono-substituted by lower alkyl, lower alkoxy or halogen; t represents 0 or 1; the mono or bis lower lower alkyl esters thereof; and pharmaceutically acceptable salts thereof.

Most preferred are the said perhydroindoles wherein $R_4$ represents hydrogen or methyl; $R_5$ represents methyl or phenethyl; and t represents 1. Further preferred are the compounds wherein the carbon atoms bearing $R_4$ and $R_5$ are in the (R)-configuration, and pharmaceutically acceptable salts thereof.

Also included are the other functional mono and bis ester and amide derivatives, especially pro-drug functional derivatives cited above. Preferred are e.g. the benzyl esters, the (methyl-, methoxy-, chloro-) substituted benzyl esters, the pyridylmethyl esters, the pivaloyloxymethyl esters, the β-(hydroxy-, acetyloxy-, methoxy-)ethoxymethyl esters, and the bornyloxycarbonylmethyl esters.

The term "lower", referred to above and hereinafter in connection with organic radicals or compounds respectively, defines such with up to 7, preferably up to 4, and advantageously but one or two carbon atoms.

Pharmaceutically acceptable salts are preferably metal or ammonium salts of said acids, more particularly alkali or alkaline earth metal salts, e.g., the sodium, potassium, magnesium or calcium salt; or advantageously easily crystallizing ammonium salts derived from ammonia or organic amines, such as mono-, di- or tri-lower (alkyl, cycloalkyl or hydroxyalkyl)-amines, lower alkylenediamines or lower (hydroxyalkyl or aralkyl)-alkylammonium bases, e.g. methylamine, diethylamine, triethylamine, dicyclohexylamine, triethanolamine, ethylenediamine, tris-(hydroxymethyl)-aminomethane or benzyltrimethylammonium hydroxide. Said basic (amino, mono- or di-lower alkylamino)- lower alkyl esters form also acid addition salts, which are preferably such of therapeutically acceptable inorganic or organic acids, such as strong metalloidic acids, for example hydrohalic, e.g. hydrochloric or hydrobromic acid; sulfuric, phosphoric, nitric or perchloric acid; aliphatic or aromatic carboxylic or sulfonic acids, e.g. formic, acetic, propionic, succinic, glycollic, lactic, malic, tartaric, citric, maleic, fumaric, hydroxymaleic, pyruvic, phenylacetic, benzoic, 4-aminobenzoic, anthranilic, 4-hydroxybenzoic, salicylic, 4-aminosalicylic, pamoic, nicotinic; methanesulfonic, ethanesulfonic, hyroxyethanesulfonic, ethylenesulfonic, halogen-benzenesulfonic, toluenesulfonic, naphthalenesulfonic, sulfanilic or cylohexylsulfamic acid; or ascorbic acid.

In addition to the pharmaceutically acceptable salts cited above, any pro-drug derivatives thereof, e.g. pharmaceutically acceptable esters and amides of the carboxylic acids of this invention that may be convertible by solvolysis or under physiological conditions to the said carboxylic acids, represent an object of this invention.

Said esters are preferably, e.g. the straight chain or branched lower alkyl esters unsubstituted or suitable substituted such as the pivaloyloxymethyl, bornyloxycarbonylmethyl, pyridylmethyl, α-carboxyethyl or suitable esterified α-carboxyethyl esters, which are prepared by methods well known to the art and exemplified herein.

Said amides are preferably, e.g. simple primary and secondary amides and amides derived from the amino acids or derivatives thereof, such as the amides derived from alanine, phenylalanine and the like.

The compounds of this invention exhibit valuable pharmacological properties, primarily hypotensive, antihypertensive and cardioactive effects, inter alia due to their angiotensin converting enzyme inhibitory activity. This is demonstrable by in vivo or in vitro animal tests, using advantageously mammals, e.g. rats, cats, dogs or isolated organs thereof, as test objects. The animals may either be normotensive or hypertensive, e.g. genetically hypertensive rats, or renal hypertensive rats and dogs, and sodium-depleted dogs. Said compounds can be applied to them enterally or parenterally, advantageously orally or intravenously, for example within gelatin capsules or in the form of starchy suspensions or aqueous solutions respectively. The applied dosage may range between about 0.01 and 100 mg/kg/day, preferably between about 0.1 and 50 mg/kg/day advantageously between about 0.1 and 25 mg/kg/day.

The in vivo lowering effect on the blood pressure is recorded either directly by means of a catheter, for example, placed in the dog's femoral artery, or indirectly by sphygmomanometry at rat's tail, and a transducer, expressing the blood pressure prior and after dosing in mm Hg.

The compounds of the invention also exhibit an inhibitory effect against the angiotensin I pressure response of normotensive rats. The enzyme renin normally causes specific hydrolysis of the circulating protein renin-substrate. This hydrolysis generates angiotensin I, which is further hydrolyzed by the action of said converting enzyme to the potent vasoconstrictor angiotensin II. The inhibition of said enzyme prevents the generation of angiotensin II from I and, therefore, attenuates any pressure response following an angiotensin I challenge.

The corresponding in vivo test is performed with male, normotensive rats, which are anesthetized with 100—120 mg/kg i.p. of sodium ethyl-(1-methylpropyl)-malonylthiourea. A femoral artery and saphenous vein are cannulated for direct blood pressure measurement and i.v. administration of angiotensin I and compounds of this invention. After the basal blood pressure is stabilized, pressor responses to 3 challenges of 0.33 µg/kg of angiotensin I i.v., in 5 minute intervals, are obtained. Such pressure response are again obtained 5, 10, 15, 30 and 60 minutes after either i.v., or p.p. administration (stomach tube) of the

compounds to be tested, and compared with the initial responses. Any observed decrease of said pressor response is an indication of anigiotenson I converting enzyme inhibition, ranging up to 80% after 10 mg/kg i.v. or lower doses, which decrease may be sustained up to 60 minutes.

The in vitro inhibition of the angiotensin-converting enzyme by the compounds of this invention can be demonstrated analogous to Biochim. Biophys. Acta *293*, 451 (1973). According to this method said compounds are dissolved at about 1 mM concentrations in phosphate buffer, externally cooled with ice. To these solutions various µl amounts of 1 mM of histidyl-leucine in phosphate buffer are added, followed by 100 µl of 5 mM hippuryl-histidyl-leucine in phosphate buffer and 50 µl of the angiotensin-converting enzyme, which is freshly prepared from lungs of adult male rabbits in Tris buffer, containing potassium and magnesium chloride, as well as sucrose. Said solutions are incubated at 37° for 30 minutes and combined with 0.75 ml of 0.6 N aqueous sodium hydroxide to stop further reaction. Then 100 µl of o-phthalaldehyde are added at room temperature, and 10 minutes later 100 µl of 6N hydrochloric acid. These samples are read against water in a spectrophotometer set at 360 nm, and the optical densities thereof estimated. They are corrected for the standard curve via a conversion factor expressing nanomoles of histidyl-leucine formed during said 30 minutes incubation period. The results are plotted against drug concentration to determine the $IC_{50}$, i.e. the drug concentration which gives half the activity of the control sample containing no drug. Again, said representative members of the compounds of this invention are very effective in this in vitro test system, down to $IC_{50}$ values as low or lower than 10 nM.

Illustrative of the invention, the $IC_{50}$ for angiotensin converting enzyme inhibition for 1-(4-carboxy-4-phenethylbutanoyl)-3aS,7aS-octahydroindole-2S-carboxylic acid is about ($1X10^{-8}M$).

Also illustrative of the invention 1-(4-carboxy-2R,4R-dimethylbutanoyl)octahydroindole-2S-carboxylic acid inhibits the pressor response folowing angiotensin I challenge by about 77 and 40% in the rat at a dose of 1.0 and 0.1 mg/kg i.v. respectively; 1-(4-carboethoxy)-2R,4R-dimethylbutanoyl)-octahydroindole-2S-carboxylic acid inhibits the pressor response by about 84 and 24% at a dose of 1.0 and 0.1 mg/kg i.v. respectively.

Accordingly, the compounds of this invention are valuable antihypertensive agents, especially useful for ameliorating hypertension (regardless of etiology) and/or heart-conditions, such as congestive heart failure, and/or other edemic or ascitic diseases, e.g. hepatic cirrhosis. They are also useful intermediates in the preparation of other valuable products, especially of corresponding pharmaceutical compositions.

The compounds of this invention are prepared according to conventional methods by

1) reducing an indoline or indole derivative corresponding to formula I, or

2) condensing a compound of formula II

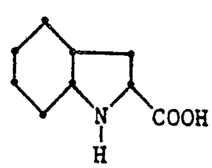

(II)

or a functional derivative thereof, with a compound of formula III

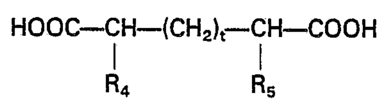

(III)

or a reactive functional derivative thereof; or with a compound of formula III, wherein the $CHR_5COOH$ group is esterified or amidized in accordance with the definition given above, or a reactive functional derivative thereof.

In the process 1) the reduction is carried out advantageously by hydrogenation in the presence of a hydrogenation catalyst, e.g. rhodium or palladium, in an inert solvent such as ethanol, at atmospheric or super-atmospheric pressure, and preferably at a temperature range of 20° to 50°.

A functional derivative of a compound of formula II is for example an ester thereof. A compound of formula III in which the group $CHR_5COOH$ is esterified, is for example in form of a lower alkyl ester.

Reactive functional derivatives of said compounds of formula III and derivatives thereof are preferably such of monocarboxylic acids, e.g. the half-ester halides and the half ester mixed anhydrides thereof, or the cyclic anhydrides of said dicarboxylic acids of formula III.

Said condensation of the compounds of formula II or functional derivatives thereof, e.g. an ester as defined above, with a said reactive functional derivative of a compound of formula III occurs either spontaneously, or in the presence of condensing agents, such as organic or inorganic bases, e.g. said salt-forming amines or alkali metal carbonates.

The condensation of preferably a functional derivative, e.g. an ester of a compound of formula II, with a said mono functional derivative of a compound of formula III having a free carboxylic acid, e.g. a half ester derivative thereof, may also be carried out in the presence of a condensing agent, e.g. a disubstituted carbodiimide, such as dicyclohexylcarbodiimide, or 1,1'-carbonyldiimidazole.

The starting materials of the process 1) are described for example in our U.S. Patent 4,374,847 and can be prepared according to methods known per se, e.g. as illustrated herein.

The starting materials of formula II and functional derivatives thereof, preferably the cis-2S-isomer thereof, may be prepared by reduction, e.g. hydrogenation of indoline-2-carboxylic acid (preferably the 2S-isomer) or indole-2-carboxylic acid and derivatives, e.g. as described in European Patent Application 37,231.

Said mono- or bis-functional derivatives of the indoline-2-carboxylic acids corresponding to the formula I or of the perhydroindole-2-carboxylic acid of formula I, wherein either or both carboxy groups are e.g. esterified by identical or different radicals may be prepared by condensing a said diacid or a mono ester derivative thereof with an esterifying agent of the formula

$$R_6—Z \qquad (IV)$$

wherein Z represents hydroxy or preferably a reactive esterified hydroxyl group; and $R_6$ represents any of the ester radicals defined hereinabove and comprising lower alkyl, e.g. methyl, ethyl, n- or i-propyl or -butyl; substituted lower alkyl, e.g. the ω-amino, ω-mono- or N-dimethylamino, α-carboxy or α-carbethoxy-(ethyl, propyl or butyl); aryl(lower)alkyl, e.g. benzyl, (methyl-, methoxy-, chloro-)substituted benzyl, or pyridylmethyl; lower alkanoyloxy(lower)alkyl, e.g. pivaloyloxymethyl; 3-phthalidyl or (methyl-, methoxy-, chloro-(substituted 3-phthalidyl, (hydroxy-; lower alkanoyloxy-, lower alkoxy-)substituted lower alkoxymethyl, e.g. β-(hydroxy-, acetyloxy-, methoxy-) ethoxymethyl; bicycloalkyloxycarbonyl-(lower)alkyl, e.g. unsubstituted or lower alkyl substituted bicyclo[2,2,1]-heptyloxycarbonyl-(lower) alkyl, advantageously bornyloxycarbonylmethyl; or 1-(lower alkoxycarbonyloxy)-(lower) alkyl, e.g. 1-(methoxy-, ethoxy- or propoxycarbonyloxy)-methyl, ethyl or propyl.

A reactive esterified hydroxyl group Z in a compound of the formula IV is a hydroxyl group esterified by a strong inorganic or organic acid. Corresponding Z groups are in particular halogen, for example chlorine, bromine or preferably iodine, also sulfonyloxy groups, such as lower alkyl- or arylsulfonyloxy groups, for example methane-, ethane-, benzene- or toluenesulfonyloxy groups.

The esterification of the carboxyl groups, optionally in salt form, with a compound of formula IV, wherein Z represents a reactive esterified hydroxyl group, is performed in a manner known per se, in the presence of for example an organic base, such as an organic amine, for example a tertiary amine, such as tri-lower-alkylamine, for example trimethylamine, triethylamine or ethyl-diisopropylamine, an N,N-di-lower-alkyl-aniline, for example N,N-dimethylaniline, a cyclic tertiary amine, such as an N-lower-alkylated morpholine, for example N-methylmorpholine, a base of the pyridine type, for example pyridine, an inorganic base, for example hydroxides, carbonates, or hydrogen carbonates of alkaki metals or alkaline-earth metals, for example sodium, potassium or calcium hydroxide, carbonate or hydrogen carbonate, or a quaternary ammonium base, such as a tetraalkylammonium hydroxide, carbonate or hydrogen carbonate, for example in which alkyl is, e.g. methyl, ethyl, propyl, isopropyl, butyl, or the like, or an oxirane, for example a lower 1,2-alkylene oxide, such as ethylene oxide or propylene oxide.

The indoline di-carboxylic acid corresponding to the formula I or the dicarboxylic acid of the formula I or a monoester thereof is preferably first converted into a salt of one of the stated organic or inorganic bases, especially into the sodium or potassium salt, and is then reacted with a compound of the formula IV. The compounds of formula IV are known or can be prepared by methods well-known to the art.

A compound of the formula IV wherein Z is a reactive esterified hydroxyl group can be prepared *in situ*. For example, a compound of the formula IV wherein Z is chloro can be converted by treatment with sodium iodide in a solvent, for example in acetone or acetonitrile, into a compound of the formula IV wherein Z is iodo; or esterification can be carried out with a chloro compound of the formula IV in the presence of sodium iodide.

The esterification reaction is performed in a suitable inert solvent or solvent mixture, for example in a carboxylic acid amide, e.g. dimethylformamide, a halogenated hydrocarbon, e.g. methylene chloride, carbon tetrachloride or chlorobenzene, a ketone, e.g. acetone, an ester, e.g. ethyl acetate, or a nitrile, e.g. acetonitrile, or mixtures thereof, preferably at room temperature, or if necessary at a reduced or elevated temperature, about at −40° to about 100°C, advantageously at −10° to +40°C, and/or in an inert-gas atmosphere, for example in a nitrogen atmosphere.

Esterification of the carboxylic acid with an alcohol of formula IV, wherein Z represents hydroxy is carried out in a manner known *per se*, preferably in the presence of an acid catalyst, e.g. sulfuric acid or boron trifluoride etherate preferably at an elevated temperature, advantageously ranging from about 40° to 100°C.

The compounds of the invention can be converted into each other according to conventional methods. Thus, for example, resulting amides or esters may be further hydrolyzed or alcoholyzed (transesterified) with inorganic acids, such as hydrohalic or sulfuric acids, in known manner; and said alcoholysis is analogously performed in the presence of both said acids and the corresponding unsubstituted or substituted lower alkanols; or with aqueous alkalies, such as alkali metal carbonates or hydroxides, respectively. Resulting free acids may be esterified with e.g. said unsubstituted or substituted lower alkanols, alkyl halides, or diazoalkanes, or converted into said metal, ammonium or acid addition salts in conventional manner.

Thus, for example, any resulting free acid or base can be converted into a corresponding metal, ammonium or acid addition salt respectively, by reacting it with an equivalent amount of the corresponding base, basic salt, acid or ion exchange preparation, e.g. said acids with alkali or ammonium hydroxides or carbonates, or said aminoalkyl esters with said inorganic or organic acids respectively. Any resulting salt may also be converted into the free compounds, by liberating the latter with stronger acids or bases respectively. In view of the close relationship between the free compounds, and the salts thereof, whenever a compound of the invention, or intermediate thereof, is referred to in this context, a corresponding salt is also intended, provided such is possible or appropriate under the circumstances.

In case mixtures of geometrical or optical isomers of the compounds of formulae I to IV are obtained, these can be separated into the single isomers by methods in themselves known, e.g., by fractional distillation, crystallization and/or chromatography. Racemic products can likewise be resolved into the optical antipodes, for example, by separation of diastereomeric salts thereof, such as according to J. Org. Chem. *43*, 3803 (1978), e.g. by the fractional crystallization of d- or l-(tartrates, mandelates, camphorsulfonates, or 1-naphthyl-1-ethylisocyanates), or of d- or l-(α-methylbenzylammonium, cinchonidine, cinchonine, quinine, quinidine, ephedrine, dehydroabietylamine, brucine or strychnine)-salts. The preferred starting material of formula II is the 2S-optical isomer (epimer) thereof.

The reactions mentioned herein are carried out according to standard methods, in the presence or absence of diluents, preferably such as are inert to the reagents and are solvents thereof, of catalysts, alkaline or acidic condensing or said other agents respectively and/or inert atmospheres, at low temperatures, room temperature or elevated temperatures, preferably at the boiling point of the solvents used, at atmospheric or superatmospheric pressure.

The pharmacologically active compounds of the invention are useful in the manufacture of pharmaceutical compositions comprising an effective amount thereof in conjunction or admixture with excipients suitable for either enteral or parenteral administration. Preferred are tablets and gelatin capsules comprising the active ingredient together with a) diluents, e.g. lactose, dextrose, sucrose, mannitol, sorbitol, cellulose and/or glycine, b) lubricants, e.g. silica, talcium, stearic acid, its magnesium or calcium salt and/or polyethyleneglycol, for tablets also c) binders, e.g. magnesium aluminium silicate, starch paste, gelatin, tragacanth, methylcullulose, sodium carboxymethylcellulose and/or polyvinylpyrrolidone, if desired, d) disintegrants, e.g. starches, agar, alginic acid or its sodium salt, or effervescent mixtures and/or e) absorbents, colorants, flavors and sweeteners. Injectable compositions are preferably aqueous isotonic solutions or suspensions; and suppositories are advantageously prepared from fatty emulsions or suspensions. Said compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances, e.g. other antihypertensive agents and/or diuretics. Said compositions are prepared according to conventional mixing, granulating or coating methods respectively, and contain about 0.12 to 75%, preferably about 1 to 50% of the active ingredient. A unit dosage for a mammal of about 50—70 kg weight may contain between about 5 and 100 mg of the active ingredient.

The following examples are intended to illustrate the invention and are not to be construed as being limitations thereon. Temperatures are given in degree Centigrade, and all parts wherever given are parts by weight. If not mentioned otherwise, all evaporations are performed under reduced pressure, preferably between about 15 and 100 mg Hg.

Example 1

A solution of 1-(4-carboxy-2R,4R-dimethylbutanoyl)indoline-2S-carboxylic acid (250 mg) in 20 ml of ethanol is hydrogenated at 3 atmospheres pressure in the presence of 100 mg of 5% rhodium on carbon at room temperature for 18 hours. The reaction is filtrated and the filtrate is evaporated to give 1-(4-carboxy-2R,4R-dimethylbutanoyl)-octahydroindole-2S-carboxylic acid, mp 83—86°, $[\alpha]_D = -95.5°$ (c=0.67 in ethanol).

The starting material is obtained as follows:

To a solution of 2.63 g of ethyl indoline-2-S-carboxylate in 40 ml of methylene chloride containing 4.8 g of anhydrous potassium carbonate is added 2.39 g of 4-carboethoxy-2R,4R-dimethylbutanoyl chloride. The reaction is stirred overnight at room temperature and then extracted with 20 ml of water. The organic layer is washed with 15 ml of 1N hydrochloric acid and 15 ml of water, dried ($Na_2SO_4$) and evaporated to give ethyl 1-(4-carboethoxy-2R,4R-dimethylbutanoyl)-indoline-2S-carboxylate as an oil having $[\alpha]_D = -130.10$ (c = 1.0 in ethanol).

To a solution of 3.4 g of ethyl 1-(4-carboethoxy-2R,4R-dimethylbutanoyl)-indoline-2S-carboxylate in 30 ml of methanol is added 28.2 ml of 1N aqueous sodium hydroxide solution. The reaction mixture is stirred at room temperature for 4 hours, evaporated to remove methanol and acidified with 3.5 ml of concentrated hydrochloric acid. The mixture is extracted four times with 10 ml of methylene chloride. The combined extract is dried over sodium sulfate and evaporated to dryness and the residue is recrystallized from ether-petroleum ether to give 1-(4-carboxy-2R,4R-dimethylbutanoyl)-indoline-2S-carboxylic acid melting at 132—134°, $[\alpha]_D = -144°$ (c = 1.0 in ethanol).

Example 2

A solution of 1-(4-carboethoxy-2R,4R-dimethylbutanoyl)-indoline-2S-carboxylic acid (1.5 g) in 100 ml of ethanol is hydrogenated at 3 atmospheres pressure in the presence of 500 mg of 5% rhodium on carbon at room temperature for 3 days. The reaction is filtered and the filtrate is evaporated to give 1-(4-carboethoxy-2R,4R-dimethylbutanoyl)-octahydroindole-2S-carboxylic acid, mp 50—53°, $[\alpha]_D = -95°$ (c = 1.04 in ethanol).

The starting material is prepared as follows:

To a solution of 43 g of indoline-2S-carboxylic acid hydrochloride in 15 ml pyridine at 0°C is added 1.35 g of 4-carboethoxy-2R,4R-dimethylbutanoyl chloride. The reaction mixture is stirred at room temperature for 3 hours and evaporated under vacuum. The residue is treated with 20 ml of 3N hydrochloric acid and extracted three times with 10 ml of methylene chloride and the extract is evaporated to dryness. The 1-(4-carboethoxy-2R,4R-dimethylbutanoyl)-indoline-2S-carboxylic acid obtained is dissolved in 75 ml of ether and treated with 2.2 ml dicyclohexylamine to yield the crystalline dicyclohexylammonium salt. This is slurried in a mixture of 40 ml of ethyl acetate and 45 ml of 5% aqueous potassium bisulfate solution for 1 hour. The ethyl acetate layer is separated, washed with water, dried over sodium sulfate, and evaporated to dryness. Crystallization from hexane yields 1-(4-carboethoxy-2R,4R-dimethylbutanoyl)-indoline-2S-carboxylic acid, melting at 125—7°, $[\alpha]_D = -159°$(c = 0.2 in ethanol).

Example 3

To a solution of 0.47 g of 3aS, 7aS-octahydroindole-2S-carboxylic acid hydrochloride in 10 ml of pyridine at 0°C is added 0.46 g of 4-carboethoxy-2R-methyl-4R-phenethylbutanoyl chloride. The reaction is stirred at room temperature for 3 hours and evaporated under vacuum. The residue is treated with 10 ml of 3N hydrochloric acid and extracted 3 times with 10 ml of methylene chloride and the extract is evaporated to dryness. The residue is crystallized from ether-hexane to give 1-(4-carboethoxy-2R-methyl-4R-phenethylbutanoyl)-3aS,7aS-octahydroindole-2S-carboxylic acid.

The preparation of 3aS,7aS-octahydroindole-2S-carboxylic acid is described in European Patent application 37,231 and Tetrahedron Letters *23*, 1677—1680 (1982).

The starting material is prepared in part according to the general process described in Tetrahedron Letters *1980*, 4233—6, as follows:

Aqueous sodium hydroxide (1 N, 25 ml) is added to a solution of 2.0 g of L-prolinol (US patent 3,935,280) in 50 ml of $CH_2Cl_2$. After cooling the reaction mixture to 0°C, 4.0 g of 4-phenylbutyric acid chloride is added and the reaction is stirred vigorously for 4 hours at 0°C, followed by 1 hour at room temperature. The reaction is diluted with an equal volume of $CH_2Cl_2$ and the layers separated. The organic phase is washed with 30 ml of water and dried over $Na_2SO_4/K_2CO_3$. The solvent is evaporated to yield 4.4 g of N-(4-phenylbutanoyl)-L-prolinol having IR peaks at 3280 and 1605 $cm^{1-}$, $[\alpha]^{25}_D = -40.3°$ (methanol). 20.5 g of N-methyl-N,N'-dicyclohexylcarbodiimidium iodide (Angew. Chem., Int. Ed. *11*, 229 (1972) is added to a solution of 5.3 g of R-(−)-3-benzyloxy-2-methylpropanol (Helv. Chim. Acta *60*, 925 (1977) in 200 ml dry tetrahydrofuran under nitrogen and the reaction is stirred at room temperature for 14 hours. The solvent is evaporated and 20 ml of ether and 5 ml of pentane are added. The resulting yellow solid is collected and the mother liquors are chromatographed on 200 g of silica gel with pentane to yield, 6.68 g of the S(+)-3-benzyloxy-2-methylpropyl iodide having Rf = 0.60 (9:1 of pentane: ether/$SiO_2$), $[\alpha]^{25}_D = +11.1°$ (MeOH). N-(4-phenylbutanoyl)-L-prolinol is dissolved in 2 ml of dry tetrahydrofuran and added dropwise to a solution of lithium disopropylamide (15.6 m moles) in 50 ml of tetrahydrofuran at 0°C under nitrogen. After 30 minutes at 0°C, 2.03 g of S-(+)-3-benzyloxy-2-methylpropyl iodide is added dropwise in 2ml of dry tetrahydrofuran. The reaction is stirred at 0°C for 5 hours, at −15°C for 15 hours and quenched at 0°C with excess saturated ammonium chloride solution. The reaction mixture is diluted with 30 ml of ether. The layers are separated and the organic phase is washed with 16 ml of 1N HCl, 15 ml of brine, 15 ml of saturated sodium bicarbonate and dried over sodium sulfate.

Evaporation of solvent yields 3.6 g of an oil which is filtered through 60 g of silica gel with ethyl acetate to yield 2.3 g of N-(R,R-5-benzyloxy-4-methyl-2-phenethylpentanoyl)-L-prolinol having Rf 0.51 (EtoAc/$SiO_2$).

A solution of 2.0 g of N-(R,R-5-benzyloxy-4-methyl-2-phenetylpentanoyl)-L-prolinol in 50 ml of 1N ethanolic hydrochloric acid is refluxed under nitrogen for 15 hours. The solvent is evaporated and the residue is chromatographed on 60 g of silica gel with pentane: ether (2:1) to yield 0.65 g of ethyl, R,R-5-benzyloxy-4-methyl-2-phenethylpentanoate having Rf 0.37 (9:1 of pentane: ether/$SiO_2$), $[\alpha]^{25}_D + 2.85$ (EtOH).

A solution of 0.6 g of ethyl R,R-5-benzyloxy-4-methyl-2-phenethylpentanoate in 50 ml of anhydrous ethanol is hydrogenated at 2.72 atm for 3 hours at room temperature with 0.5 g of 5% palladium on charcoal catalyst. The catalyst is then removed by filtering through celite and the solvent is evaporated to yield 0.41 g of ethyl R,R-5-hydroxy-4-methyl-2-phenethylpentanoate having Rf = 0.36 (1:1 of pentane-ether).

Ethyl R,R-5-hydroxy-4-methyl-2-phenethylpentanoate (0.35 g) is dissolved in 15 ml of dry dimethylformamide at room temperature under nitrogen. Pyridinium dichromate (2.5 g) is added and the reaction mixture is stirred for 15 hours at room temperature before being poured into 150 ml of water. The aqueous solution is extracted with ether (4 × 40 ml). The ethereal extracts are washed with 30 ml of water and then three times with 20 ml of a 1:1 solution of sodium bicarbonate: potassium carbonate (pH = 10.5). The basic

wash is acidified to pH = 2 with concentrated sulfuric acid, while keeping the temperature between 5 and 10°C, and extracted with ether (4 × 20 ml). The ethereal extracts are washed with 20 ml of brine and dried over $Na_2SO_4/MgSO_4$. Evaporation of the solvent yields 0.28 g of 4-carboethoxy-2R-methyl-4R-(phenethyl)-butanoic acid, having Rf = 0.50 (99:1:100 of ether:AcOH:hexane), $[\alpha]^{25}_D = -4.91°$ (EtOh).

Treatment of 4-carboethoxy-2R-methyl-4R-(phenethyl)-butanoic acid with oxalyl chloride in methylene chloride yields 4-carboethoxy-2R-methyl-4R-phenethyl-butanoyl chloride.

Example 4

To a solution of 0.5 g of 3aS,7aS-octahydroindole-2S-carboxylic acid in 5 ml of pyridine is added 0.55 g of 2-phenethyl-glutaric anhydride. The mixture is heated at 80° under an atmosphere of nitrogen for 15 hours, concentrated, and partitioned between 20 ml of ethyl acetate and 10 ml of 1N hydrochloric acid. The organic layer is separated, washed with water, dried over sodium sulfate, filtered and concentrated to give a foam. The residue is dissolved in 5 ml of diethyl ether and triturated with hexane. The solid is collected, dried at 35°/1 mm Hg to give 1-(4-carboxy-4-phenethylbutanoyl)3aS,7aS-octahydroindole-2S-carboxylic acid melting at 60—63°; $[\alpha]_D = -28.9°$ (c = 1.0 in methanol).

The starting material is obtained as follows:

A solution of 10.0 g of indoline-2S-carboxylic acid hydrochloride in 80 ml of absolute ethanol is hydrogenated at 3 atmospheres pressure in the presence of 5% rhodium on carbon catalyst at room temperature for 24 hours. The resulting mixture is filtered free of catalyst and evaporated to dryness. The foam is treated with hot ethyl acetate. The solid is collected, dried under high vacuum to give 3aS,7aS-octahydroindole-2S-carboxylic acid hydrochloride melting at 136—9°; $[\alpha]_D = -21.7°$ (c=1 in ethanol).

In a similar fashion 1-[4-carboxy-4-(3-phenylpropyl)-butanoyl]-3aS,7aS octahydroindole-2S-carboxylic acid, 1-[4-carboxy-4-(4-phenylbutyl)-butanoyl]-octahydroindole-2S-carboxylic acid, and 1-[4-carboxy-4-(6-phenylhexyl)-butanoyl]-3aS,7aS-octahydroindole-2S-carboxylic acid are prepared using 2-(3-phenylpropyl)glutaric anhydride, 2-(4-phenylbutyl)-glutaric anhydride, and 2-(6-phenylhexyl)-glutaric anhydride as starting materials, respectively.

Condensation of ethyl octahydroindole-2S-carboxylate with 4R-carboethoxy-6-phenylhexanoic acid e.g. in methylene chloride in the presence of triethylamine and 1-(3-dimethylaminopropyl)-3-ethylcarbodiimide hydrochloride and selective hydrolysis with 2,21N aqueous potassium hydrochloride solution yields 1-(4-carboethoxy-4R-phenethylbutanoyl)-octahydroindole-2S-carboxylic acid.

The starting material is prepared as follows:

8.10 g of N-(4-phenylbutanoyl)-L-prolinol in 50 ml of tetrahydrofuran are added to 71 mMol of lithium diisopropylamide in 270 ml of tetrahydrofuran at −20°. After 1 hour 3.1 ml of allyl bromide are added. After 1 hour at −20° the reaction mixture is diluted with 250 ml of diethyl ether. The layers are separated and the organic phase is washed with 200 ml of 0.5N aqueous hydrochloric acid and 200 ml of saturated aqueous sodium chloride solution, dried over sodium sulfate, and evaporated to give the N-(2R-allyl-4-phenylbutanoyl)-L-proplinol having $[\alpha]_D = -23.5°$ (c = 1 in methanol).

A solution of 7.65 g of N-(2R-allyl-4-phenyl butanoyl)-L-prolinol in 350 ml of 10% ethanolic sulfuric acid is refluxed for 10 hours. The solvent is evaporated and the residue is partitioned between 100 ml of water and 200 ml of diethyl ether. The layers are separated and the organic phase is washed with 50 ml of saturated aqueous sodium bicarbonate solution, dried over sodium sulfate and evaporated to give the ethyl 2R-allyl-4-phenyl-butyrate having $R_f = 0.42$ (9:1 hexane: ether/$SiO_2$).

To 22.2 ml of 1M borane in tetrahydrofuran at 0°C is added 3.1 g of 2-methyl-2-butene. After 2 hours at 0°, 4.76 g of ethyl 2R-allyl-4-phenyl-butyrate are added, After 2 hours 7.3 ml of 3N aqueous sodium hydroxide solution and 7.3 ml of 30% aqueous hydrogen peroxide are added. After 30 minutes the reaction mixture is diluted with 75 ml of diethyl ether. The layers are separated and the aqueous layer is extracted with 2 × 25 ml of diethyl ether. The combined ether extracts are washed with 25 ml of 1% aqueous sodium carbonate solution and 25 ml of saturated aqueous sodium sulfite solution. The organic phase is dried over magnesium sulfate and evaporated to give ethyl 5-hydroxy-2R-phenethyl-pentanoate with NMR peaks at 7.3, 4.25, 3.61, 1.55—2.78 and 1.27 ppm.

To 4.97 g of ethyl 5-hydroxy-2R-phenethyl-pentanoate in 100 ml of dimethylformamide is added 37.4 g of pyridinium dichromate. The reaction mixture is stirred overnight at room temperature and then poured into 300 ml of ice water. The mixture is extracted with 5 × 100 ml of diethyl ether. The combined organic portions are washed with 100 ml of 5% aqueous sodium carbonate solution. The aqueous phase is acidified with concentrated sulfuric acid to pH 2 and extracted with 3 × 100 ml of diethyl ether. These ether extracts are dried over magnesium sulfate and evaporated to give 4R-carboethoxy-6-phenyl-hexanoic acid with $[\alpha]_D = +4.32°$ (c = 1 in methanol).

Example 5

According to the methods illustrated in the previous examples are prepared the following 2S,3aS,7aS-octahydroindoles of formula I wherein $R_4$ is hydrogen and t is 1.

| Compound | $R_5$ | (R)-$CHR_5COOH$ derivative |
|---|---|---|
| a | $(CH_2)_3C_6H_5$ | — |
| b | $(CH_2)_3C_6H_5$ | ethyl ester |
| c | $(CH_2)_4C_6H_5$ | — |
| d | $(CH_2)_4C_6H_5$ | ethyl ester |
| e | $(CH_2)_6C_6H_5$ | — |
| f | $(CH_2)_6C_6H_5$ | ethyl ester |

*Starting materials*: preparation by condensation according to example 4.
4R-carboethoxy-7-phenylheptanoic acid,
4R-carboethoxy-8-phenylheptanoic acid,
4R-carboethoxy-9-phenylheptanoic acid.

Example 6

Preparation of 10,000 tablets each containing 10 mg of the active ingredient of Example 2:

| | |
|---|---|
| 1-(4-carboethoxy-2R,4R-dimethylbutanoyl) octahydroindole-2S-carboxylic acid | 100.00 g |
| Lactose | 1,107,00 g |
| Corn starch | 75.00 g |
| Polyethylene glycol 6,000 | 75.00 g |
| Talcum powder | 75.00 g |
| Magnesium stearate | 18.00 g |
| Purified water | q.s. |

Procedure:

All the powders are passed through a screen with openings of 0.6 mm. Then the drug substance, lactose, talcum, magnesium stearate and half of the starch are mixed in a suitable mixer. The other half of the starch is suspended in 40 ml of water and the suspension added to the boiling solution of the polyethylene glycol in 150 ml of water. The paste formed is added to the powders which are granulated, if necessary, with an additional amount of water. The granulate is dried overnight at 35°, broken on a screen with 1.2 mm openings and compressed into tablets using concave punches with 6.4 mm diameter, uppers bisected.

Prepared similarly to the previous examples are tablets and capsules containing other octahydroindoles of formula I and functional derivatives thereof, e.g. those given in the other examples.

Example 7

Prepared according to the following examples herein for the corresponding derivatives of indoline 2S-carboxylic acid are the following esters and amides of 1-(4-carboxy-4-phenethylbutanoyl)-3aS, 7aS-octahydroindole-2S-carboxylic acid (formula I, t=1, $R_4$=H, $5_5$=phenethyl).

| —CH(CH$_2$CH$_2$C$_6$H$_5$)CO— | Octahydroindole-2-CO— |
|---|---|
| pivaloyloxymethoxy | OH |
| 1-bornyloxycarbonylmethoxy | OH |
| L-2-acetoxymethylpyrrolidino | OH |
| L-2-hydroxymethylpyrrolidino | OH |
| β-methoxyethoxymethoxy | OH |
| 3-phthalidoxy | OH |
| benzyloxy | OH |
| ethoxy | L-phenylalaninome ester |
| OH | L-phenylalanino |
| β-carboethoxyethoxy | OH |
| 3-pyridylmethoxy | OH |
| β-(carbobenzyloxyamino)ethyl-amino | OC$_2$H$_5$ |
| β-(carbobenzyloxyamino)ethylamino | OH |
| β-aminoethylamino | OH |
| benzyloxy | pivaloyloxymethoxy |
| OH | pivaloyloxymethoxy |
| carboethoxymethylamino | OC$_2$H$_5$ |
| carboxymethylamino | OH |
| 1-(ethoxycarbonyloxy)ethoxy | OH |

## Example 8

To a solution of 1.43 g of indoline-2S-carboxylic acid hydrochloride in 15 ml pyridine at 0°C is added 1.35 g of 4-carboethoxy-2R,4R-dimethylbutanoyl chloride. The reaction mixture is stirred at room temperature for 3 hours and evaporated under vacuum. The residue is treated with 20 ml of 3N hydrochloric acid and extracted three times with 10 ml of methylene chloride and the extract is evaporated to dryness. The 1-(4-carboethoxy-2R,4R-dimethylbutanoyl)-indoline-2S-carboxylic acid obtained is dissolved in 75 ml of ether and treated with 2.2 ml dicyclohexylamine to yield the crystalline dicyclohexylammonium salt. This is slurried in a mixture of 40 ml of ethyl acetate and 45 ml of 5% aqueous potassium bisulfate solution for 1 hour. The ethyl acetate layer is separated, washed with water, dried over sodium sulfate, and evaporated to dryness. Crystallization from hexane yields 1-(4-carboethoxy-2R,4R-dimethylbutanoyl)-indoline-2S-carboxylic acid, melting at 125—7°, $[\alpha]_D$ = 159° (c = 0.2 in ethanol).

## Example 9

A solution of 0.45 g of ethyl indoline-2S-carboxylate and 0.30 g of 2R,4R-dimethylglutaric anhydride in 10 ml of toluene is stirred at 70° for 18 hours. The reaction is cooled to room temperature, washed twice with 5 ml of 1N hydrochloric acid and extracted twice with 10 ml of 5% sodium bicarbonate. The combined bicarbonate portions are acidified with 4.0 ml of 12N hydrochloric acid and extracted three times with 10 ml of methylene chloride. The combined methylene chloride portions are dried over $Na_2SO_4$ and evaporated to give ethyl 1-(4-carboxy-2R,4R-dimethyl-butanoyl)-indoline-2S-carboxylate as an oil having NMR peaks at 1.0 to 1.3, 4.15, 5.10, 7.2 and 8.4 ppm.

## Example 10

To a solution of 5.0 g of ethyl indoline-2S-carboxylate hydrochloride and 2.2 g of triethylamine in 40 ml of toluene are added 3.1 g of (2R,4R)-dimethylglutaric anhydride. The reaction mixture is stirred at 80° for 4 hours, then cooled to room temperature and washed with 10 ml of 1N hydrochloric acid and 10 ml of water. The organic layer is dried over sodium sulfate and evaporated. The residue is crystallized from diethyl etherhexane to give ethyl 1-(4-carboxy-2R,4R-dimethylbutanoyl)-indoline-2S-carboxylate melting at 110—112°, $[\alpha]_D$ = −156.5° (c = 0.2 in ethanol).

## Example 11

To 1.7 g of 4-(pivaloyloxymethoxycarbonyl)-2R,4R-dimethylbutanoyl chloride in 25 ml of pyridine at room temperature is added indoline-2S-carboxylic acid hydrochloride (1.2 g). The reaction is stirred 3 hours at room temperature and then evaporated. The residue is dissolved in 75 ml of saturated aqueous sodium bicarbonate and washed with 2 × 50 ml of ether. The aqueous layer is acidified with 10 ml of 2N aqueous hydrochloric acid and extracted with 3 × 75 ml of methylene chloride. The combined methylene chloride layer is dried over magnesium sulfate and evaporated to give 1-[4-(pivaloyloxymethoxy-carbonyl)-2R,4R-dimethylbutanoyl]-indoline-2S-carboxylic acid, m.p. 109—112°C, $[\alpha]_D = -136.2°$ (c = 0.94 in ethanol), NMR ($CDCl_3$): 1.2, 1.8, 2.5, 3.35, 5.1, 5.75, 7.2 8.3 9.4 ppm, IR ($CHCl_3$): 3500—3200, 2950, 1740 $cm^{-1}$.

The starting material is prepared as follows: A mixture of 5.0 g of 2R,4R-dimethylglutaric anhydride and 3.65 ml of benzyl alcohol is stirred at 85° for 3 hours to give 4-(carbobenzyloxy)-2R,4R-dimethylbutanoic acid. NMR ($CDCl_3$): 1.2, 1.8, 2.6, 5.2, 7.4, 11.2 ppm.

The above acid (3.3 g) is treated with 5.9 ml of 2.21N potassium hydroxide. The solution is evaporated. Toluene (100 ml) is added and the mixture is evaporated to give the potassium 4-(carbobenzyloxy)-2R,4R-dimethylbutanoate.

To chloromethyl pivalate (2.56 g) in 50 ml of acetone is added sodium iodide (2.54 g). The reaction is stirred at room temperature for 3 hours. The reaction is filtered and the filtrate is evaporated. To the residue in 25 ml of dimethylformamide is added potassium 4-(carbobenzyloxy)-2R,4R-dimethylbutanoate (3.7 g) in 25 ml of dimethylformamide. The reaction is stirred at room temperature for 18 hours and then evaporated. The residue is dissolved in 150 ml of ether and washed with 3 × 50 ml of 10% aqueous sodium bicarbonate and 3 × 50 ml of saturated aqueous sodium chloride. The organic layer is dried over magnesium sulfate and evaporated to give pivaloyloxymethyl-4-(carbobenzyloxy)-2R,4R-dimethylbutanoate as an oil, NMR ($CDCl_3$): 1.2, 1.8, 2.5, 5.2, 5.8, 7.45 ppm.

A solution of 5.3 g of pivaloyloxymethyl-4-(carbobenzyloxy)-2R,4R,dimethylbutanoate in 150 ml of ethanol is hydrogenated at atmospheric pressure in the presence of 0.5 g of 10% palladium on carbon. The reaction is filtered and evaporated to give 4-(pivaloyloxymethoxycarbonyl)-2R,4R-dimethylbutanoic acid, NMR ($CDCl_3$): 1.2, 1.7, 2.55, 5.8, 10.3 ppm.

To 1.7 g of the above 4-(pivaloyloxymethoxycarbonyl)-2R,4R-dimethylbutanoic acid in 30 ml of methylene chloride at room temperature is added 1.7 ml of oxalyl chloride. The reaction is stirred at room temperature for 2.5 hours and then evaporated to give 4-(pivaloyloxymethoxycarbonyl)-2R,4R-dimethylbutanoyl chloride, used directly in the above condensation, NMR ($CDCl_3$): 1.2, 1.9, 2.8, 5.85 ppm.

## Example 12

To 6.5 g of 4-(l-bornyloxycarbonylmethoxycarbonyl)-2R,4R-dimethylbutanoic acid in 100 ml of methylene chloride is added 9.0 ml of oxalyl chloride. After stirring 3 hours at room temperature the reaction is evaporated. The residue is dissolved in 70 ml of pyridine and indoline-2S-carboxylic acid hydrochloride (3.7 g) is added. The reaction is stirred 2.5 hours at room temperature. The reaction is evaporated. The residue is dissolved in 75 ml of saturated aqueous sodium bicarbonate and washed with 3 × 50 ml of ether. The aqueous layer is acidified with 10 ml of 6N hydrochloric acid and extracted with 3 × 75 ml of methylene chloride. The combined methylene chloride portions are dried over magnesium sulfate and evaporated to give 1-[4-(l-bornyloxycarbonylmethoxycarbonyl)-2R,4R-dimethylbutanoyl]-indoline-2S-carboxylic acid, m.p. 65—67° with $[\alpha]_D = -139°$ (c = 0.915 in ethanol, NMR ($CDCl_3$): 3.4, 4.9, 7.1—8.4 ppm, IR ($CHCl_3$): 3500—3100, 2950, 1735 $cm^{-1}$.

The starting material is prepared as follows: A mixture of 5.0 g of l-borneol and 7.74 ml of chloroacetyl chloride is stirred at 100° for 3 hours and then evaporated to give l-bornyl chloroacetate as an oil, NMR ($CDCl_3$): 0.88, 0.91, 1.1—2.5, 4.1, 5.0 ppm.

To 5.0 g of the above l-bornyl chloroacetate in 70 ml of acetone is added sodium iodide (3.4 g). The reaction is stirred 3 hours at room temperature and then filtered and evaporated to give l-bornyl iodoacetate, which is used directly in the next step.

l-Bornyl iodoacetate (6.7 g) potassium 4-(carbobenzyloxy)-2R,4R-dimethylbutanoate (6.1 g) are combined in 100 ml of dimethyl formamide. The reaction is stirred at room temperature overnight and then evaporated. The residue is dissolved in 200 ml of ether and washed with 2 × 50 ml of saturated aqueous sodium bicarbonate and 50 ml of brine. The organic layer is dried over magnesium sulfate and evaporated to give l-bornyloxycarbonylmethyl-4-(carbobenzyloxy)-2R,4R-dimethylbutanoate, NMR ($CDCl_3$): 4.6, 5.1, 7.4 ppm.

l-Bornyloxycarbonylmethyl 4-(carbobenzyloxy)-2R,4R-dimethylbutanoate (8.0 g) in 125 ml of ethanol is hydrogenated at one atmosphere in the presence of 0.8 g of 10% palladium on carbon. The reaction is filtered and evaporated to give 4-(l-bornyloxycarbonymethoxycarbonyl)-2R,4R-dimethylbutanoic acid; NMR ($CDCl_3$): 4.6, 5.0, 8.2 ppm.

## Example 13

4-[(L-2-Acetoxymethylpyrrolidino)-carbonyl]-2R,4R-dimethylbutanoic acid (3.6 g) in 7 ml of oxalyl chloride is stirred at room temperature for 3 hours and then evaporated. The acid chloride is dissolved in 50 ml of pyridine and indoline-2S-carboxylic acid hydrochloride (2.76 g) is added. The reaction is stirred for 2 hours at room temperature and then is evaporated. The residue is dissolved in 50 ml of saturated

aqueous sodium bicarbonate and washed with 3 × 50 ml of ether. The aqueous layer is acidified with 10 ml of 6N aqueous hydrochloric acid and extracted with 3 × 50 ml of methylene chloride. The combined methylene chloride portions are dried over magnesium sulfate and evaporated to give 1-(4-[L-2-acetoxymethylpyrrolidino)-carbonyl]-2R,4R-(dimethylbutanoyl)-indoline-2S-carboxylic acid, m.p. 109—112°, $[\alpha]_D = -172.4°$ (c = 1.03 in ethanol), NMR ($CDCl_3$): 1.2, 4.9, 7.1, 8.3, 9.2 ppm, IR ($CHCl_3$): 3500—3100, 2950, 1730 $cm^{-1}$.

The starting material is prepared as follows:

To 4-(Carbobenzyloxy)-2R,4R-dimethylbutanoic acid (3.9 ml) in 50 ml of methylene chloride is added 4.1 ml of oxalyl chloride. The reaction is stirred at room temperature for 3 hours and then evaporated to give 4-(carbobenzyloxy)-2R,4R-dimethylbutanoyl chloride which is used directly in the next step.

To a mixture of L-prolinol (1.42 g) in 35 ml of methylene chloride and 17.7 ml of 1N aqueous sodium hydroxide at 0° is added the above acid chloride (4.3 g). The reaction is stirred for 4 hours at 0° and then 1 hour at room temperature. The layers are separated and the aqueous layer is washed with 2 × 35 ml of methylene chloride. The combined organic portions are washed with 2 × 40 ml of 2N aqueous hydrochloric acid, dried with magnesium sulfate and evaporated to give benzyl 4-[L-2-hydroxy-methylpyrrolidino)-carbonyl]-2R,4R-dimethylbutanoate, NMR ($CDCl_3$): 1.05, 1.2, 5.1, 7.35 ppm.

The above amide (4.5 g) is stirred in 3 ml of acetyl chloride at reflux for 3 hours. The reaction is then evaporated to give benzyl 4-[L-2-acetoxymethylpyrrolidino)-carbonyl)-2R,4R-dimethylbutanoate, NMR ($CDCl_3$): 1.07, 1.15, 2.0, 5.17, 7.4 ppm.

Benzyl 4-[(L-2-acetoxymethylpyrrolidino)-carbonyl]-2R,4R-dimethylbutanoate (4.6 g) in 100 ml of ethanol is hydrogenated at one atmosphere in the presence of 0.5 g of 10% palladium on carbon. The reaction is filtered and the filtrate is evaporated to give 4-[L-2-acetoxymethylpyrrolidino)-carbonyl]-2R,4R-dimethylbutanoic acid, NMR ($CDCl_3$): 1.2, 2.03 ppm.

Example 14

1-(4-[L-(2-acetoxymethylpyrrolidino)-carbonyl)-2R,4R-dimethylbutanoyl)-indoline-2S-carboxylic acid (1.5 g) in 100 ml of methanol is added 1.2 g of potassium carbonate. The reaction is stirred overnight at room temperature. The reaction is evaporated and the residue in 150 ml of methylene chloride is acidified with 75 ml of 2N aqueous hydrochloric acid. The layers are separated and the organic layer is washed with 75 ml of brine, dried over magnesium sulfate and then evaporated to give 1-(4-[N-(L-2-hydroxymethylpyrrolidino)-carbonyl]-2R,4R-dimethylbutanoyl)-indoline-2S-carboxylic acid, m.p. 95—99°, $[\alpha]_D = -179.3°$ (c = 0.98 in ethanol), NMR ($CDCl_3$): 1.1, 4.2, 4.9 ppm, IR ($CHCl_3$): 3500—3100, 2950, 1730 $cm^{-1}$.

Example 15

To 1,1′-carbonyldiimidazole (1.14 g) in 20 ml of methylene chloride at 0° is added 4-(β-methoxyethoxy-methoxycarbonyl)-2R,4R-dimethylbutanoic acid (1.6 g) in 20 ml of methylene chloride. After 1 hour indoline-2S-carboxylic acid hydrochloride (1.3 g) in 10 ml of pyridine is added dropwise over 10 minutes. The reaction is stirred at room temperature overnight. The reaction is evaporated. The residue is dissolved in 75 ml of methylene chloride and washed with 2 × 25 ml of 2N aqueous hydrochloric acid. The organic layer is dried over magnesium sulfate and evaporated to give crude product. The product is dissolved in 20 ml of ether, 20 ml of ethyl acetate and 0.46 ml of dicyclohexylamine are added, followed by 25 ml of pentane. The product was collected by filtration to give 1-(4-(β-methoxyethoxymethoxycarbonyl)-2R,4R-dimethylbutanoyl]-indoline-2S-carboxylic acid as the dicyclohexylammonium salt, m.p. 159—161°, $[\alpha]_D = -79.6°$ (c = 1.13, EtOH).

The starting material is prepared as follows: To 4 g of 4-(carbobenzyloxy)-2R,4R-dimethylbutanoic acid in 60 ml of ether at 0° is added 1.8 g of potassium *t*-butoxide. After 30 minutes β-methoxyethoxymethyl chloride (1.82 g) is added. The reaction is allowed to warm to room temperature over 1 hour and then stirred an additional 2 hours at room temperature. The reaction is poured into 50 ml of saturated aqueous sodium bicarbonate and extracted with 3 × 50 ml of ether. The combined ether portions are dried (magnesium sulfate) and evaporated to give β-methoxyethoxymethyl-4-(carbobenzyloxy)-2R,4R-dimethyl-butanoate as an oil, $[\alpha]_D = -32.6°$ (c = 1.19 in ethanol), NMR: 1.80, 3.35, 5.16, 5.30 ppm.

A solution of the above ester (0.5 g) in 25 ml of ethanol is hydrogenated at 1 atmosphere pressure and room temperature in the presence of 50 mg of 10% palladium on carbon. After 2 hours the reaction is filtered. The filtrate is evaporated to give 4-(β-methoxyethoxymethoxycarbonyl)-2R,4R-dimethylbutanoic acid as an oil, $[\alpha]_D = -25.7°$ (c = 0.975 in ethanol); NMR: 1.20, 1.83, 3.40, 5.40 ppm.

Example 16

To 4-(3-phthalidoxycarbonyl)-2R,4R-dimethylbutanoyl chloride (2.5 g) in 45 ml of pyridine is added indoline-2S-carboxylic acid hydrochloride (1.3 g). The reaction is stirred at room temperature for 3 hours. The reaction is evaporated and the residue in 100 ml of methylene chloride is washed with 2 × 50 ml of water. The combined aqueous portions are adjusted to pH 1 with concentrated aqueous hydrochloric acid and extracted with 3 × 25 ml of methylene chloride. The combined methylene chloride portions are washed with 2 × 25 ml of 2N aqueous hydrochloric acid, dried over magnesium sulfate and evaporated to give a foam, which is crystallized from ether-pentane to give 1-[4-(3-phthalidoxycarbonyl)-2R,4R-dimethyl-butanoyl]-indoline-2S-carboxylic acid, m.p. 119—122°; $[\alpha]_D = -104.9°$ (c = 0.65, EtOH).

The starting material is prepared as follows: A solution of 4-(carbobenzyloxy)-2R,4R-dimethylbutanoic acid (0.3 g) in 6 ml of 2N aqueous potassium hydroxide is evaporated to dryness. The residue is twice evaporated with 20 ml of toluene. To the residue in 50 ml of dimethylformamide is added 3-bromophthalide (2.55 g). The reaction is stirred overnight at room temperature. The reaction is evaporated. The residue in 40 ml of ether is washed with 2 × 20 ml of saturated aqueous sodium bicarbonate. The ether layer is dried over magnesium sulfate and evaporated to give benzyl 4-(3-phthalidoxycarbonyl)-2R,4R-dimethylbutanoate as an oil, NMR ($CDCl_3$) 1.85, 2.60, 3.50, 5.15 ppm; IR (film) 3035, 2978, 1775, 1755, 1730, 1468 $cm^{-1}$.

The above ester (3.5 g) in 45 ml of ethanol is hydrogenated at 1 atmosphere pressure and room temperature in the presence of 350 mg of 10% palladium on carbon. After 3 hours the reaction is filtered. The filtrate is evaporated to give 4-(3-phthalidoxycarbonyl)-2R,4R-dimethylbutanoic acid, $[\alpha]_D = -21.3°$ (c = 0.78 in ethanol); NMR 1.2, 1.8, 2.65, 3.70 ppm.

To 4-(3-phthalidoxycarbonyl)-2R,4R-dimethylbutanoic acid (0.30 g) in 10 ml of methylene chloride is added 0.24 ml of oxalyl chloride. The reaction is stirred at room temperature for 3 hours and then evaporated to give 4-(3-phthalidoxycarbonyl)-2R,4R-dimethylbutanoyl chloride, $[\alpha]_D = -16.1°$ (c = 0.53 in chloroform); NMR 1.3, 1.9, 2.76, 4.3 ppm.

Example 17

To indoline-2S-carboxylic acid hydrochloride (2.23 g) in 30 ml of pyridine at room temperature is added 4-(carbobenzyloxy)-2R,4R-dimethylbutanoyl chloride (3.3 g). After stirring for 3 hours at room temperature the reaction is evaporated. The residue is dissolved in 100 ml of saturated aqueous sodium bicarbonate and washed with 50 ml of ether. The aqueous layer is adjusted to pH 1 with concentrated hydrochloric acid and extracted with 3 × 50 ml of methylene chloride. The combined methylene chloride portions are stirred over florisil, dried over magnesium sulfate and evaporated to give 1-[4-(carbobenzyloxy)-2R,4R-dimethylbutanoyl]-indoline-2S-carboxylic acid, m.p. 150—153°, $[\alpha]_D = -139.1°$ (c = 0.79 in ethanol).

The starting material is prepared as follows: The above acid chloride is prepared by stirring 3.0 g of 4-(carbobenzyloxy)-2R,4R-dimethylbutanoic acid and oxalyl chloride (1.05 ml) in 50 ml of methylene chloride for 3 hours at room temperature and then evaporating the solvent to give the 4-(carbobenzyloxy)-2R,4R-dimethylbutanoyl chloride, used directly in the preceding condensation.

Example 18

To L-phenylalanine methyl ester hydrochloride (1.3 g) and 1-(4-carboethoxy-2R,4R-dimethylbutanoyl)-indoline-2S-carboxylic acid (2.0 g) in 50 ml of methylene chloride at room temperature is added 0.84 ml of triethylamine and 1.3 g of 1-(3-dimethylaminopropyl)-3-ethylcarbodiimide hydrochloride. The reaction is stirred at room temperature for 20 hours. The reaction is washed with 3 × 25 ml of 2N aqueous hydrochloric acid, 2 × 25 ml of saturated aqueous sodium bicarbonate and 25 ml of brine. The organic layer is dried over magnesium sulfate and evaporated to give N-[1-(4-carboethoxy-2R,4R-dimethylbutanoyl)-indolinyl-2S-carbonyl]-L-phenylalanine methyl ester, m.p. 110—113°, $[\alpha]_D = -119.7°$ (c = 0.75 in ethanol).

Example 19

To 2.4 g of N-[1-(4-carboethoxy-2R,4R-dimethylbutanoyl)-indolinyl-2S-carbonyl]-L-phenylalanine methyl ester is 35 ml of ethanol is added 9 ml of 1N sodium hydroxide and 10 ml of water. The reaction is stirred overnight at room temperature. The ethanol is evaporated and the aqueous solution is washed with 10 ml of ether and then acidified to pH 1 with 2N aqueous hydrochloric acid. The solution is extracted with 3 × 30 ml of ethyl acetate. The combined ethyl acetate portions are dried over magnesium sulfate and evaporated to give N-[1-(4-carboxy-2R,4R-dimethylbutanoyl)-indolinyl-2S-carbonyl]-L-phenylalanine, m.p. 161—164°; $[\alpha]_D = -104.3°$ (c = 1.15 in ethanol).

Example 20

A mixture of the acid chloride obtained from 1.8 g of 4-(α-carboethoxy-ethoxycarbonyl)-2R,4R-dimethylbutanoic acid, and indoline-2S-carboxylic acid hydrochloride (1.3 g) in 25 ml of pyridine is stirred at room temperature for 3 hours and then evaporated. The residue in 50 ml of saturated aqueous sodium bicarbonate is washed with 2 × 30 ml of ether. The aqueous layer is acidified to pH 1 with solid potassium bisulfate and extracted with 2 × 30 ml of methylene chloride. The combined methylene chloride portions are stirred over florisil and dried with magnesium sulfate and evaporated to give an oil. Trituration with 10 ml of ether gives 1-[4-(α-carboethoxy-ethoxycarbonyl)-2R,4R-dimethylbutanoyl]-indoline-2S-carboxylic acid, m.p. 151—154°, $[\alpha]_D = -146.8°$ (c = 1.06 in ethanol).

The starting material is prepared as follows: A mixture of 2R,4R-dimethylglutaric anhydride (1.0 g) and ethyl l-lactate (0.809 ml) is heated at 80° for 4 days. The crude 4-(α-carboethoxy-ethoxycarbonyl)-2R,4R-dimethylbutanoic acid is used directly, $R_F = 0.7$ (silica gel-ethyl acetate), NMR ($CDCl_3$) 5.05, 4.28, 2.58, 1.79, 1.16 ppm.

The above acid (1.8 g) in 30 ml of methylene chloride is stirred with 2 ml of oxalyl chloride for 3 hours at room temperature. Evaporation of the solvent gives 4-(α-carboethoxy-ethoxycarbonyl)-2R,4R-dimethylbutanoyl chloride as used above.

Example 21

To the acid chloride, obtained from 2.5 g of 2R,4R-dimethylglutaric anhydride as described below, in 40 ml of pyridine is added indoline-2S-carboxylic acid hydrochloride (3.2 g). The reaction is stirred at room temperature and then evaporated. The residue in 50 ml of water is washed with 3 × 50 ml of methylene chloride. The aqueous layer is adjusted to pH 3.0 with 2N aqueous hydrochloric acid and extracted with 3 × 50 ml of ethyl acetate. The combined ethyl acetate portions are dried over magnesium sulfate and evaporated. The residue is triturated with 50 ml of ether to give the crude 1-[4-(3-pyridylmethoxycarbonyl)-2R,4R-dimethylbutanoyl]-indoline-2S-carboxylic acid which is dissolved in 30 ml of 3N aqueous hydrochloric acid and washed with 30 ml of ethyl acetate. The aqueous layer is evaporated and the residue dried in vacuo at 50° to give 1-[4-(3-pyridylmethoxycarbonyl)-2R,4R-dimethylbutanoyl]-indoline-2S-carboxylic acid hydrochloride, m.p. 86—89°, $[\alpha]_D = -75.2°$ (c = 0.8 in ethanol).

The starting material is prepared as follows: A mixture of 2R,4R-dimethylglutaric anhydride (2.5 g) and 3-pyridylcarbinol (1.7 ml) are stirred at 90° for 6 hours. The reaction is cooled to give the crude 4-(3-pyridylmethoxycarbonyl)-2R,4R-dimethylbutanoic acid, NMR ($CDCl_3$), 8.83, 7.86, 7.44, 5.19, 2.58, 1.75, 1.18 ppm, IR (film) 2980, 2935, 1735, 1465, 1163 $cm^{-1}$.

The above acid in 50 ml of methylene chloride is stirred with 4.5 ml of oxalyl chloride with 3 hours at room temperature. Evaporation of the solvent gives the acid chloride, used without further purification.

Example 22

To ethyl 1-(4-carboxy-2R,4R-dimethylbutanoyl)-indoline-2S-carboxylate (2.5 g) and β-carbobenzyloxyaminoethylamine (1.6 g) in 50 ml of methylene chloride is added 1.05 ml of triethylamine and 1.6 g of 1-(3-dimethylaminopropyl)-3-ethylcarbodiimide. The reaction is stirred for 2 days at room temperature and then refluxed 4 hours. The reaction is washed with 2 × 25 ml of 2N aqueous hydrochloric acid and 2 × 25 ml of saturated aqueous sodium bicarbonate. The methylene chloride layer is dried over magnesium sulfate and evaporated to give ethyl 1-[4-(β-carbobenzyloxyamino-N-ethylcarbamoyl)-2R,4R-dimethylbutanoyl]-indoline-2S-carboxylate after trituration with ethyl ether; $R_F = 0.4$ (silica gel-ethyl acetate), NMR ($CDCl_3$) 7.41, 5.11, 4.13, 1.15 ppm.

Example 23

To ethyl 1-[4-(β-carbobenzyloxyamino-N-ethylcarbamoyl)-2R,4R-dimethylbutanoyl]-indoline-2S-carboxylate (2.4 g) in 25 ml of ethanol is added 5.2 ml of 1N sodium hydroxide in 10 ml of water. The reaction is stirred 4 hours at room temperature. The reaction is evaporated to remove ethanol. The aqueous residue is diluted to 100 ml with water and washed with 2 × 10 ml of ether, acidified to pH 1 with solid potassium bisulfate and extracted with 3 × 25 ml of methylene chloride. The combined methylene chloride extracts are dried with magnesium sulfate and evaporated. The residue is slurried in 40 ml of ether to give 1-[4-(β-carbobenzyloxyamino-N-ethylcarbamoyl)-2R,4R-dimethylbutanoyl]-indoline-2S-carboxylic acid, m.p. 104—106°, NMR ($CDCl_3$) 8.39, 7.36, 7.2, 6.15, 5.02 ppm.

Example 24

A solution of 0.82 g of 1-[4-(β-carbobenzyloxyamino-N-ethylcarbamoyl)-2R,4R-dimethylbutanoyl]-indoline-2S-carboxylic acid in 35 ml of ethanol is hydrogenated at 1 atmosphere pressure in the presence of 70 mg of 10% palladium on carbon for 3 hours at room temperature. The reaction is filtered and the filtrate is evaporated to give 1-[4-(β-amino-N-ethylcarbamoyl)-2R,4R-dimethylbutanoyl]-indoline-2S-carboxylic acid, m.p. 202—204°, $[\alpha]_D = -99.5°$ (c = 0.86 in ethanol).

Example 25

To 1.9 g of the potassium salt of 1-[4-(carbobenzyloxy)-2R,4R-dimethylbutanoyl]-indoline-2S-carboxylic acid in 35 ml of dimethylformamide at room temperature is added 1.15 g of iodomethyl pivalate. The reaction is stirred overnight at room temperature. The reaction is evaporated and the residue is taken up in 150 ml of ether and washed with 50 ml of saturated aqueous sodium bicarbonate and 50 ml of brine. The organic layer is dried (magnesium sulfate) and evaporated to give pivaloyloxymethyl-[4-(carbobenzyloxy)-2R,4R-dimethylbutanoyl]-indoline-2S-carboxylate, NMR ($CDCl_3$) 1.20, 3.20, 5.70, 7.25 ppm; $R_f = 0.8$ (90:9:1, $CHCl_3$: EtOH: HOAc, silica gel).

Example 26

A solution of 0.60 g of pivaloyloxymethyl 1-[4-(carbobenzyloxy)-2R,4R-dimethylbutanoyl]-indoline-2S-carboxylate in 30 ml of ethanol is hydrogenated at room temperature and one atmosphere pressure in presence of 50 mg of 10% palladium on carbon. The reaction is filtered and the filtrate is evaporated to give pivaloyloxymethyl 1-[4-carboxy-2R,4R-dimethylbutanoyl]-indoline-2S-carboxylate, m.p. 143—145°, $[\alpha]_D = -139.6°$ (c = 0.9 in ethanol).

Example 27

To 2.4 g of ethyl 1-(4-carboxy-2R,4R-dimethylbutanoyl)-indoline-2S-carboxylate in 100 ml of methylene chloride is added 1.0 g of glycine ethyl ester hydrochloride, 1.0 ml of triethylamine and 1.5 g of 1-(3-dimethylaminopropyl)-3-ethylcarbodiimide hydrochloride. The reaction is stirred overnight at room

temperature. The reaction is washed with 50 ml of 2N aqueous hydrochloric acid and 50 ml of saturated aqueous sodium bicarbonate. The organic layer is dried over magnesium sulfate and evaporated to give ethyl 1-[4-(carboethoxymethylcarbamoyl)-2R,4R-dimethylbutanoyl]-indoline-2S-carboxylate, $[\alpha]_D = -83.6°$ (c = 0.78 in ethanol), NMR ($CDCl_3$) 1.30, 4.20, 5.20, 7.25 ppm.

Example 28

To 2.3 g of ethyl 1-[4-carboethoxymethylcarbamoyl)-2R,4R-dimethylbutanoyl]-indoline-2S-carboxylate in 40 ml of ethanol at room temperature is added 6.0 ml of 2N aqueous potassium hydroxide followed by 15 ml of water. The reaction is stirred at room temperature for 3 hours. The ethanol is removed by evaporation and the aqueous portion is washed with 2 × 10 ml of ether. The aqueous layer is adjusted to pH 1 by addition of solid potassium bisulfate and extracted with 3 × 25 ml of methylene chloride. The combined methylene chloride portions are dried over magnesium sulfate and evaporated to give 1-[4-(carboxymethylcarbamoyl)-2R,4R-dimethylbutanoyl]-indoline-2S-carboxylic acid, m.p. 70—73°, $[\alpha]_D = -86.5°$ (c = 0.75 in ethanol).

Example 29

To a solution of 4-[1-(ethoxycarbonyloxy)-ethoxycarbonyl]-2R,4R-dimethylbutanoic acid (5.65 g) in 100 ml of methylene chloride at 0°C is added 1,1'-carbonyldiimidazole (3.3 g). After 1 hour indoline-2S-carboxylic acid hydrochloride (4.1 g) and triethylamine (4.1 g) are added and the reaction is stirred at room temperature overnight. The reaction is diluted with 100 ml of methylene chloride and washed with ice-cooled 50 ml of water containing 4 ml of 12 N hydrochloric acid. The aqueous layer is washed with 2 × 50 ml of methylene chloride. The combined organic portions are dried ($MgSO_4$) and evaporated to give 1-{4-[1-(ethoxycarbonyloxy)-ethoxycarbonyl]-2R,4R-dimethylbutanoyl}-indoline-2S-carboxylic acid.

The starting material is prepared as follows: A solution of 1-chloroethyl ethyl carbonate (5.50 g) and sodium iodide (5.95 g) in 75 ml of acetone is refluxed for 1 hour. The acetone is removed in vacuo and the residue is partitioned between 100 ml of ether and 100 ml of water. The ether layer is washed with 25 ml of 5% aqueous sodium metabisulfite and dried ($MgSO_4$). The ether is removed in vacuo. The remaining ethyl 1-iodoethyl carbonate is immediately dissolved in 15 ml of dimethylformamide and added to a solution of potassium 4-(carbobenzyloxy)-2R,4R-dimethyl-butanoate (10.4 g) in 50 ml of dimethylformamide. The reaction is stirred at room temperature for 18 hours and then evaporated. The residue is dissolved in 150 ml of ether and washed with 3 × 50 ml of 10% aqueous sodium bicarbonate. The ether layer is dried ($MgSO_4$) and evaporated to give 1-(ethoxycarbonyloxy)-ethyl-4-(carbobenzyloxy)-2R,4R-dimethylbutanoate.

A solution of the above benzyl ester (7.9 g) in 150 ml of ethanol is hydrogenated at atmospheric pressure in the presence of 0.5 g of 10% palladium on carbon. The reaction is filtered and evaporated to give 4-[1-(ethoxycarbonyloxy)-ethoxycarbonyl]-2R,4R-dimethylbutanoic acid.

Example 30

A solution of 2.0 g of l-bornyl iodoacetate and 2.30 g of the potassium salt of ethyl 1-(4-carboxy-2R,4R-dimethylbutanoyl)-indoline-2S-carboxylate in 30 ml of dimethylformamide is stirred at room temperature overnight and then evaporated. The residue is dissolved in 100 ml of ether and washed with 2 × 25 ml of water. The organic layer is dried over magnesium sulfate and evaporated to give ethyl 1-[4-(l-bornyloxy-carbonylmethoxycarbonyl)-2R,4R-dimethylbutanoyl]-indoline-2S-carboxylate.

**Claims**

1. A compound of the formula I

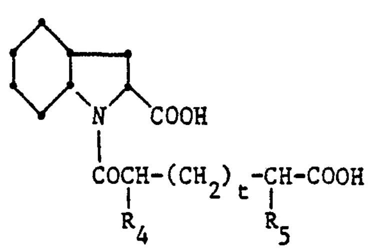

(I)

wherein $R_4$ represents hydrogen or lower alkyl; $R_5$ represents lower alkyl; or $R_5$ represents lower alkyl substituted by aryl in which aryl represents phenyl or phenyl substituted by lower alkyl, lower alkoxy, trifluoromethyl or halogen; t represents 0 or 1; the unsubstituted amides and mono- or di-lower alkylamides thereof; lower alkylene amides thereof, wherein the lower alkylene group together with the amide nitrogen forms a 5-, 6- or 7-membered ring, or said lower alkylene amides substituted on the ring by hydroxy-(lower)alkyl or by lower alkanoyloxy-(lower)alkyl; α-(lower)-carboalkoxy- or α-carboxy-substituted lower alkylamides thereof; α-(lower)carboalkoxy- or α-carboxy-substituted aryl-(lower) alkylamides in which aryl represents phenyl or 3-indolyl; (amino or acylamino)-(lower) alkylamides thereof; lower alkyl esters thereof; (amino, mono- or di-lower alkylamino, carboxy or lower carboalkoxy)-substituted lower alkyl esters thereof; aryl-(lower) alkyl esters thereof in which aryl represents phenyl or pyridyl; lower

alkanoyloxy-(lower) alkyl esters thereof; phthalidyl esters thereof; (hydroxy, lower alkanoyloxy, or lower alkoxy)-substituted (lower) alkoxymethyl esters thereof; bicycloalkyloxycarbonyl-(lower) alkyl esters thereof having up to 10 carbon atoms in the bicycloalkyl group; lower alkoxycarbonyloxy-(lower) alkyl esters, wherein "lower" defines a group with up to 7 carbon atoms, or pharmaceutically acceptable salts thereof.

2. A compound as claimed in claim 1 wherein t is 1, or pharmaceutically acceptable salts thereof.

3. A compound as claimed in claim 1, wherein $R_4$ represents hydrogen or $C_{1-4}$-alkyl; $R_5$ represents $C_{1-4}$-alkyl; or $R_5$ represents $C_{1-4}$-alkyl substituted by aryl in which aryl represents phenyl or phenyl mono-substituted by lower alkyl, lower alkoxy or halogen; t represents 0 or 1; wherein "lower" defines a group with up to 7 carbon atoms, the mono or bis lower lower alkyl esters, or pharmaceutically acceptable salts thereof.

4. A compound as claimed in claim 3 wherein t is 1, or pharmaceutically acceptable salts thereof.

5. A compound as claimed in claim 3, in which $R_4$ represents hydrogen or methyl; $R_5$ is methyl or phenethyl; t represents 1; a mono lower alkyl ester, wherein "lower" defines a group with up to 7 carbon atoms, or pharmaceutically acceptable salts thereof.

6. 1-(4-Carboxy-2R,4R-dimethylbutanoyl)-octahydroindole-2S-carboxylic acid or pharmaceutically acceptable salts thereof.

7. 1-(4-Carboethoxy-2R,4R-dimethylbutanoyl)-octahydroindole-2S-carboxylic acid or pharmaceutically acceptable salts thereof.

8. 1-(4-Carboxy-4-phenethylbutanoyl)-3aS,7aS-octahydroindole-2S-carboxylic acid or pharmaceutically acceptable salts thereof.

9. A pharmaceutical preparation comprising a compound claimed in any one of claims 1 to 8, in admixture or conjunction with a pharmaceutically suitable carrier.

10. A compound named in any one of claims 1 to 8 for use in a therapeutic method of treating humans and animals.

11. A compound named in any one of claims 1 to 8 as hypotensive, antihypertensive and cardioactive agent.

12. The use of a compound of any one of claims 1 to 8 in producing of a pharmaceutical preparation.

13. Process for the manufacture of a compound of the formula

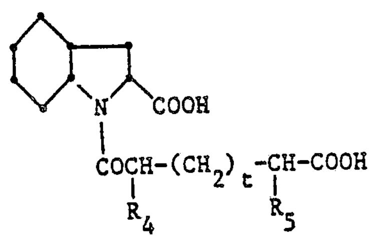

(I)

wherein $R_4$, $R_5$ and t have the meaning, as given in claim 1, esters and amines thereof as defined in claim 1 or salts thereof, which consists in

1) reducing an indoline or indole derivative corresponding to formula I, or

2) condensing a compound of formula II

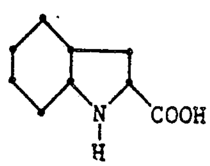

(II)

or functional derivative thereof, with a compound of formula III

$$HOOC-\underset{R_4}{\underset{|}{CH}}-(CH_2)_t-\underset{R_5}{\underset{|}{CH}}-COOH \qquad (III)$$

or a reactive functional derivative thereof; or with a compound of formula III, wherein the $CHR_5COOH$ group is esterified or amidized as defined in claim 1, or a reactive functional derivative thereof, and, if desired, converting any resulting compound into another compound of the invention, and/or, if required, converting a resulting free compound into a salt or a resulting salt into the free compound or into another salt, and, if required, resolving a mixture of isomers or racemates obtained into the single isomers or racemates, and, if required, resolving a racemate obtained into the optical antipodes.

14. The compounds obtainable according to claim 13.

**Patentansprüche**

1. Eine Verbindung der Formel I,

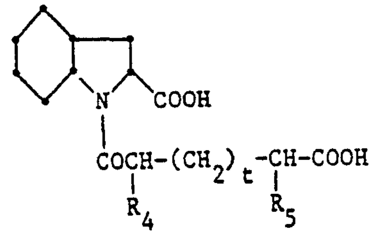

(I)

worin $R_4$ Wasserstoff oder Niederalkyl bedeutet; $R_5$ für Niederalkyl oder für durch Aryl substituiertes Niederalkyl steht, wobei Aryl Phenyl oder durch Niederalkyl, Niederalkoxy, Trifluormethyl oder Halogen substituiertes Phenyl bedeutet; worin t Null oder eins bedeutet; die unsubstituierten Amide und Mono- oder Diniederalkylamide davon; Niederalkylenamide davon, worin die Niederalkylengruppe zusammen mit dem Amidstickstoff einen 5-, 6- oder 7-gliedrigen Ring bildet, oder besagte Niederalkylenamide, die im Ring durch Hydroxyniederalkyl oder durch Niederalkanoyloxyniederalkyl substituiert sind; α-Niedercarboalkoxy- oder α-Carboxy-substituierte Niederalkylamide davon; α-Niedercarboalkoxy- oder α-Carboxy-substituierte Arylniederalkylamide, in denen Aryl Phenyl oder 3-Indolyl bedeutet; (Amino oder Acylamino)-Niederalkylamide davon; Niederalkylester davon; durch Amino, Mono- oder Diniederalkylamino, Carboxy oder Niedercarboalkoxy substituierte Niederalkylester davon; Arylniederalkylester davon, in denen Aryl Phenyl oder Pyridyl bedeutet; Niederalkanoyloxy-niederalkylester davon; Phthalidylester davon; durch Hydroxy, Niederalkanoyloxy oder Niederalkoxy substituierte Niederalkoxymethylester davon; Bicycloalkyloxycarbonylniederalkylester davon mit bis zu 10 Kohlenstoffatomen in der Bicycloalkylgruppe, Niederalkoxycarbonyloxyniederalkylester davon; worin der Begriff "Nieder" jeweils eine Gruppe mit bis zu sieben Kohlenstoffatomen bezeichnet, oder pharmazeutisch annehmbare Salze davon.

2. Eine Verbindung nach Anspruch 1, worin t für eins steht, oder pharmazeutisch annehmbare Salze davon.

3. Eine Verbindung nach Anspruch 1, worin $R_4$ Wasserstoff oder $C_{1-4}$-Alkyl bedeutet; $R_5$ für $C_{1-4}$-Alkyl oder für durch Aryl substituiertes $C_{1-4}$-Alkyl steht, wobei Aryl Phenyl oder durch Niederalkyl, Niederalkoxy oder Halogen monosubstituiertes Phenyl bedeutet; worin t Null oder eins bedeutet; worin der Begriff "Nieder" jeweils eine Gruppe mit bis zu sieben Kohlenstoffatomen bezeichnet, die Mono- oder Bisniederalkylester, oder die pharmazeutisch annehmbaren Salze davon.

4. Eine Verbindung nach Anspruch 3, worin t eins bedeutet, oder pharmazeutisch annehmbare Salze davon.

5. Eine Verbindung nach Anspruch 3, worin $R_4$ Waserstoff oder Methyl bedeutet; $R_5$ für Methyl oder Phenethyl steht; worin t eins bedeutet; einen Mononiederalkylester davon, worin der Begriff "Nieder" eine Gruppe mit bis zu sieben Kohlenstoffatomen bezeichnet, oder pharmazeutisch annehmbare Salze davon.

6. 1-(4-Carboxy-2R,4R-dimethylbutanoyl)octahydroindol-2S-carbonsäure oder pharmazeutisch annehmbare Salze davon.

7. 1-(4-Carboethoxy-2R,4R-dimethylbutanoyl)octahydroindol-2S-carbonsäure oder pharmazeutisch annehmbare Salze davon.

8. 1-(4-Carboxy-4-phenethylbutanoyl)-3aS,7aS-octahydroindol-2S-carbonsäure oder pharmazeutisch annehmbare Salze davon.

9. Ein pharmazeutisches Präparat enthaltend eine Verbindung nach einem der Ansprüche 1 bis 8 im Gemisch oder verbunden mit einem pharmazeutisch geeigneten Träger.

10. Eine Verbindung nach einem der Ansprüche 1 bis 8 zur Verwendung in einem therapeutischen Verfahren zur Behandlung von Menschen oder Tieren.

11. Eine Verbindung nach einem der Ansprüche 1 bis 8 als hypotensives, antihypertensives und kardioaktives Mittel.

12. Die Verwendung einer Verbindung nach einem der Ansprüche 1 bis 8 zur Herstellung eines pharmazeutischen Präparates.

13. Verfahren zur Herstellung einer Verbindung der Formel

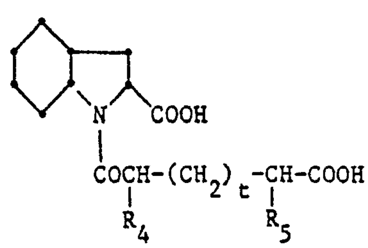

(I)

worin $R_4$, $R_5$ und t die in Anspruch 1 angegebene Bedeutung aufweisen, von Estern und Amiden davon, wie in Anspruch 1 definiert, oder von Salzen davon, dadurch gekennzeichnet, dass man

1) ein Indolin- oder ein Indolderivat, das der Formel I entspricht, reduziert, oder

2) eine Verbindung der Formel II

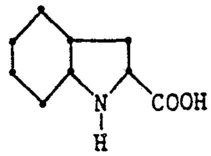

(II)

oder ein funktionelles Derivat davon mit einer Verbindung der Formel III

$$HOOC—CH(R_4)—(CH_2)_t—CH(R_5)—COOH \quad \text{(III)}$$

oder einem reaktiven funktionellen Derivat davon oder mit einer Verbindung der Formel III, worin die $CHR_5COOH$-Gruppe verestert oder amidiert ist, wie in Anspruch 1 definiert, oder einem reaktiven funktionellen Derivat davon, kondensiert, und, wenn erwünscht, eine erhaltene Verbindung in eine andere Verbindung der Erfindung umwandelt, und/oder, wenn erforderlich, eine erhaltene freie Verbindung in ein Salz oder ein erhaltenes Salz in die freie Verbindung oder in ein anderes Salz umwandelt, und, wenn erforderlich, ein erhaltenes Gemisch von Isomeren oder Racematen in die einzelnen Isomere oder Racemate auftrennt, und, wenn erforderlich, ein erhaltenes Racemat in die optischen Antipoden auftrennt.

14. Die nach Anspruch 13 erhältlichen Verbindungen.

**Revendications**

1. Un composé de formule I

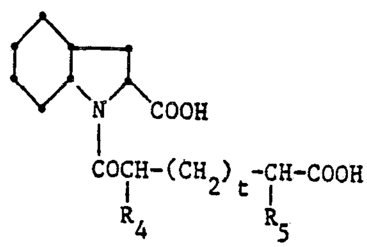

(I)

dans laquelle $R_4$ représente un hydrogène ou un alkyle inférieur; $R_5$ représente un alkyle inférieur; ou $R_5$ représente un alkyle inférieur substitué par un aryle, l'aryle représentant un phényle ou un phényle substitué par un alkyle inférieur, un alcoxy inférieur, un trifluorométhyle ou un halogène; t représente 0 ou 1; les amides non substitués et les mono- ou di-alkylamides inférieurs de celui-ci; les alkylène-amides inférieurs de celui-ci dans lesquels le groupe alkylène inférieur avec l'azote d'amide forme un cycle à 5, 6 ou 7 chaînons, ou lesdits alkylène-amides inférieurs substitués sur le cycle par un hydroxy-alkyle (inférieur) ou un alcanoyloxy inférieur -alkyle(inférieur); les alkylamides inférieurs α-carbalcoxy(inférieur)- ou α-carboxy-substitués de celui-ci; les aryl-alkyl(inférieur)-amides α-carbalcoxy inférieur- ou α-carboxy-substitués dans lesquels l'aryle représente un phényle ou un 3-indolyle; les (amino ou acylamino)-alkylamides inférieurs de celui-ci; les esters alkyliques inférieurs de celui-ci; les esters alkyliques inférieurs (amino, mono- ou di-alkylamino inférieur, carboxy ou carbalcoxy inférieur)-substitués de celui-ci; les esters aryl-alkyliques inférieurs où l'aryle représente un phényle ou un pyridyle de celui-ci; les esters alcanoyloxy(inférieur)-alkyliques inférieurs de celui-ci; les esters phtalidyliques de celui-ci; les esters alcoxyméthyliques-(inférieurs) (hydroxy, alcanoyloxy inférieur ou alcoxy inférieur)-substitués de celui-ci; les esters bicyclo-alkoxycarbonyl-alkyliques(inférieurs) de celui-ci ayant jusqu'à 10 atomes de carbone dans le groupe bicycloalkyle; les esters alcoxy(inférieur)-carbonyloxyalkyliques inférieurs, où "inférieur" qualifie un groupe ayant jusqu'à 7 atomes de carbone, ou leurs sels pharmaceutiquement acceptables.

2. Un composé comme revendiqué dans la revendication 1 où t est 1 ou ses sels pharmaceutiquement acceptables.

3. Un composé comme revendiqué dans la revendication 1 où $R_4$ représente un hydrogène ou un alkyle en $C_{1-4}$; $R_5$ représente un alkyle en $C_{1-4}$; ou $R_5$ représente un alkyle en $C_{1-4}$ substitué par un aryle où l'aryle représente un phényle ou un phényle mono-substitué par un alkyle inférieur, un alcoxy inférieur ou un halogène; t représente 0 ou 1; où "inférieur" qualifie un groupe ayant jusqu'à 7 atomes de carbone; ses esters mono- ou bis-alkyliques inférieurs ou leurs sels pharmaceutiquement acceptables.

4. Un composé comme revendiqué dans la revendication 3, où t est 1 ou ses sels pharmaceutiquement acceptables.

5. Un composé comme revendiqué dans la revendication 3 où $R_4$ représente un hydrogène ou un méthyle; $R_5$ est un méthyle ou un phénéthyle; t représente 1; un ester monoalkylique inférieur de celui-ci, où "inférieur" qualifie un groupe ayant jusqu'à 7 atomes de carbone; ou leurs sels pharmaceutiquement acceptables.

6. Acide 1-(4-carboxy-2R,4R-diméthylbutanoyl)-octahydro-indole-2S-carboxylique ou ses sels pharmaceutiquement acceptables.

7. Acide 1-(4-carbéthoxy-2R,4R-diméthylbutanoyl)-octahydro-indole-2S-carboxylique ou ses sels pharmaceutiquement acceptables.

8. Acide 1-(4-carboxy-4-phénéthylbutanoyl)-3aS,7aS-octahydro-indole-2S-carboxylique ou ses sels pharmaceutiquement acceptables.

9. Une préparation pharmaceutique comprenant un composé revendiqué dans l'une quelconque des revendications 1 à 8 en mélange ou en association avec un support pharmaceutiquement approprié.

10. Un composé indiqué dans l'une quelconque des revendications 1 à 8 pour l'emploi dans un procédé thérapeutique de traitement des êtres humains et des animaux.

11. Un composé indiqué dans l'une quelconque des revendications 1 à 8 comme agent hypotenseur, antihypertenseur et cardio-actif.

12. L'emploi d'un composé de l'une quelconque des revendications 1 à 8 dans la production d'une préparation pharmaceutique.

13. Procédé pour la fabrication d'un composé de formule

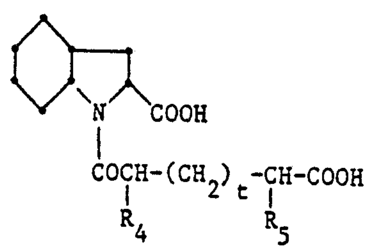

(I)

dans laquelle $R_4$, $R_5$ et t ont la signification indiquée dans la revendication 1, ses esters et ses amides comme défini dans la revendication 1 ou leurs sels, qui consiste à

1) réduire un dérivé d'indoline ou d'indole correspondant à la formule I, ou

2) condenser un composé de formule II

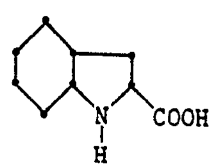

(II)

ou un dérivé fonctionnel de celui-ci, avec un composé de formule III

$$HOOC-\underset{R_4}{CH}-(CH_2)_t-\underset{R_5}{CH}-COOH \qquad (III)$$

ou un dérivé fonctionnel réactif de celui-ci; ou avec un composé de formule III où le groupe $CHR_5COOH$ est estérifié ou amidé comme défini dans la revendication 1, ou un dérivé fonctionnel réactif de celui-ci et, si on le désire, transformer tout composé obtenu en un autre composé de l'invention et/ou, s'il le faut, transformer un composé libre obtenu en un sel ou un sel obtenu en le composé libre ou en un autre sel et, s'il le faut, dédoubler un mélange d'isomères ou de racémates obtenu en les isomères ou racémates isolés et, s'il le faut, dédoubler un racémate obtenu en les atipodes optiques.

14. Les composés pouvant être obtenus selon la revendication 13.